(19) Europäisches Patentamt
European Patent Office
Office européen des brevets

(11) **EP 0 901 357 B1**

(12) **EUROPÄISCHE PATENTSCHRIFT**

(45) Veröffentlichungstag und Bekanntmachung des Hinweises auf die Patenterteilung:
**05.07.2000 Patentblatt 2000/27**

(51) Int Cl.⁷: **A61F 13/02**, C09J 5/08, C09J 7/02, C08J 9/12, C09J 7/04

(21) Anmeldenummer: **97921804.7**

(22) Anmeldetag: **26.04.1997**

(86) Internationale Anmeldenummer:
**PCT/EP97/02178**

(87) Internationale Veröffentlichungsnummer:
**WO 97/43993 (27.11.1997 Gazette 1997/51)**

(54) **PARTIELL SELBSTKLEBEND BESCHICHTETES TRÄGERMATERIAL, VERFAHREN ZU DESSEN HERSTELLUNG UND VERWENDUNG**

AIR-PERMEABLE SUBSTRATE MATERIAL PARTIALLY COATED WITH A SELF-ADHESIVE SUBSTANCE, PROCESS FOR ITS PRODUCTION AND ITS USE

MATERIAU SUPPORT AVEC COUCHE PARTIELLEMENT AUTOCOLLANTE, SON PROCEDE DE FABRICATION ET SON UTILISATION

(84) Benannte Vertragsstaaten:
**AT DE ES FR GB IT NL SE**

(30) Priorität: **18.05.1996 DE 19620107**

(43) Veröffentlichungstag der Anmeldung:
**17.03.1999 Patentblatt 1999/11**

(73) Patentinhaber: **Beiersdorf Aktiengesellschaft 20245 Hamburg (DE)**

(72) Erfinder:
• **HIMMELSBACH, Peter**
  **D-21614 Buxtehude (DE)**
• **JAUCHEN, Peter**
  **D-22455 Hamburg (DE)**

(56) Entgegenhaltungen:
**DE-A- 3 741 194      FR-A- 2 318 914**
**US-A- 5 389 168**

**Beschreibung**

Die Erfindung betrifft selbstklebend ausgerüstete Trägermaterialien, welche auf mindestens einer Seite nicht vollflächig mit einer Heißschmelzselbstklebemasse beschichtet sind, ein Verfahren zu deren Herstellung sowie ihre Verwendung.

Heißschmelzselbstklebemassen auf Basis natürlicher und synthetischer Kautschuke und anderer synthetischer Polymere sind bekannt und werden zunehmend eingesetzt. Ihr wesentlicher Vorteil besteht darin, daß im Gegensatz zu dem aus Lösung oder als wässrige Dispersion aufgebrachten Massen, keine aufwendigen und zum Teil umweltbelastenden Verfahren zum Entfernen der Lösungsmittel oder des Wassers notwendig sind.

Es ist ferner bekannt, derartige Selbstklebemassen nicht nur vollflächig sondern auch rasterpunktförmig, beispielsweise durch Siebdruck (DE-PS 42 37 252), wobei die Klebstoffpünktchen auch unterschiedlich groß und/oder unterschiedlich verteilt sein können (EP-PS 353 972), oder durch Tiefdruck in Längs- und Querrichtung zusammenhängenden Stegen aufzubringen (DE-PS 43 08 649).

Der Vorteil des rasterförmigen Auftrags besteht darin, daß die Klebematerialien bei entsprechend porösem Trägermaterial luft- und wasserdampfdurchlässig sowie in der Regel leicht wieder ablösbar sind.

Ein Nachteil dieser Produkte besteht jedoch darin, daß bei zu hoher Flächendeckung der an sich undurchlässigen Klebeschicht die Luft- und Wasserdampfdurchlässigkeit sich entsprechend verringert sowie der Klebemassenverbrauch steigt und bei geringer Flächendeckung der Klebeschicht die Klebeeigenschaften leiden, d.h. das Produkt löst sich zu leicht vom Untergrund.

Aufgabe der Erfindung war es deshalb, diese Nachteile zu vermeiden und ein Produkt bzw. Verfahren zu entwickeln, welches - bei entsprechend porösem Trägermaterial - eine sehr gute Luft- und Wasserdampfdurchlässigkeit sowie im allgemeinen gute Klebeeigenschaften bei geringem Klebemassenverbrauch aufweist.

Gelöst wird diese Aufgabe durch ein selbstklebend ausgerüstetes Trägermaterial mit einer mindestens auf einer Seite nicht vollflächig aufgebrachten Heißschmelzklebemasse, das dadurch gekennzeichnet ist, daß die Klebemasse geschäumt ist.

Die nicht vollflächig aufgebrachte geschäumte Selbstklebeschicht kann dabei rasterförmig, vorzugsweise in Kalottenform durch Siebdruck, vorliegen, oder auch in einem anderen Muster wie Gitter, Streifen, Zickzacklinien und beispielsweise auch durch Tiefdruck aufgebracht sein. Ferner kann sie beispielsweise auch aufgesprüht sein, was ein mehr oder weniger unregelmäßiges Auftragsbild ergibt.

Die Selbstklebemasse kann gleichmäßig auf dem Trägermaterial verteilt sein, sie kann aber auch funktionsgerecht für das Produkt über die Fläche unterschiedlich stark oder dicht aufgetragen sein.

Je nach Trägermaterial und dessen Temperaturempfindlichkeit kann die Selbstklebeschicht direkt aufgetragen sein oder zuerst auf einen Hilfsträger aufgebracht und dann auf den endgültigen Träger transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Der prozentuale Anteil, der mit geschäumter Selbstklebemasse beschichteten Fläche sollte mindestens 20% betragen und kann bis zu ca. 95% reichen, für spezielle Produkte bevorzugt 40-60% und 70 bis 95%. Das Flächengewicht der aufgetragenen Klebemasse sollte dabei mindestens 15g/m$^2$ betragen, um ausreichende Klebewerte zu erhalten, bevorzugt 30 - 160 g/m$^2$ in Abhängigkeit vom Trägereinsatz.

Die Klebemassen werden bevorzugt mit inerten Gasen wie Stickstoff, Kohlendioxid, Edelgasen, Kohlenwasserstoffen oder Luft oder deren Gemischen geschäumt. In manchen Fällen kann sich auch ein Schäumen durch thermische Zersetzung gasentwickelnder Substanzen wie Azo-, Carbonat- und Hydrazid-Verbindungen als geeignet erweisen.

Der Schäumungsgrad, d.h. der Gasanteil, sollte mindestens etwa 10 Vol-% betragen und kann bis zu etwa 80% reichen. In der Praxis haben sich Werte von 30-70%, bevorzugt 50% Gasanteil gut bewährt. Wird bei relativ hohen Temperaturen von ca. 100° C und vergleichsweise hohem Innendruck gearbeitet, entstehen offenporige Klebstoffschaumschichten, die besonders gut luft- und wasserdampfdurchlässig sind. Die rasterpunktförmige Beschichtung unterstützt die Luftdurchlässigkeitswerte in weiten Grenzen.

Wird auf diese Eigenschaft kein besonderer Wert gelegt, können auch durch Änderung der Parameter geschlossenporige Schaumstrukturen erzeugt und verwendet werden.

Als Selbstklebemassen lassen sich die bekannten thermoplastischen Heißschmelzklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich. Ihr Erweichungspunkt sollte höher als 80° C liegen, da die Applikationstemperatur in der Regel mindestens 90° C beträgt, bevorzugt zwischen 120° und 150° C bzw. 180-220° C bei Silikonen. Gegebenenfalls kann eine Nachvernetzung durch UV- oder Elektronenstrahlen-Bestrahlung angebracht sein.

Besonders geeignet haben sich Selbstklebemassen auf Basis von A-B-A-Blockcopolymeren, welche aus harten und weichen Segmenten bestehen. Bevorzugt ist A ein Polymerblock basierend auf Styrol und B ein Polymerblock auf Basis von Ethylen, Propylen, Butylen, Butadien, Isopren oder deren Mischungen wie Ethylen/Butylen. Zusätzlich enthalten derartige Heißschmelzklebemassen in der Regel ein oder mehrere aliphatische oder aromatische Kohlenwasserstoffharze als Klebharze, ein oder mehrere mittel- oder langkettige Fettsäuren oder deren Ester sowie Stabilisatoren und gegebenenfalls andere Hilfsstoffe. Die Mengenbereiche der Bestandteile bewegen sich meist zwischen 15-70% Blockcopolymeren, 20-70% Klebharzen, 10-50% Weichmachern und geringen Mengen Stabilisatoren und anderen Hilfsstoffen.

Als Trägermaterialien können praktisch alle Träger, die üblicherweise für technische oder medizinische Zwecke verwendet werden, eingesetzt werden, d.h. Gewebe oder Gewirke, elastisch oder unelastisch, Kunststoff- oder Metallfolien, Papiere, Vliese, Schaumstoffe oder Laminate daraus.

Die selbstklebend ausgerüsteten Trägermaterialien, die erfindungsgemäß mit einer geschäumten Heißschmelzselbstklebemasse partiell beschichtet sind, zeichnen sich durch eine Reihe von Vorteilen aus. Durch die Schäumung der Klebemasse und die dadurch entstandenen Poren in der Masse sind bei Verwendung eines an sich porösen Trägers auch die mit Klebemasse beschichteten Bereiche gut wasserdampf- und luftdurchlässig. Die benötigte Klebemassenmenge wird erheblich reduziert ohne Beeinträchtigung der Klebeeigenschaften. Die Klebemassen weisen eine überraschend hohe Anfaßklebrigkeit (tack) auf, da pro Gramm Masse mehr Volumen und damit Klebeoberfläche zum Benetzen des zu beklebenden Untergrundes zur Verfügung steht und die Plastizität der Klebemassen durch die Schaumstruktur erhöht ist. Auch die Verankerung auf dem Trägermaterial wird dadurch verbessert. Außerdem verleiht die geschäumte Klebebeschichtung den Produkten ein weiches und anschmiegsames Anfühlen.

Die subjektiven Produktvorteile Anfaßklebrigkeit und Anschmiegsamkeit lassen sich unter Verwendung einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen konstanten Temperatur der Schmelzhaftkleber zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient ($Q = \tan \delta$) zwischen dem Verlust- (G" viskoser Anteil) und dem Speichermodul (G' elastischer Anteil) ermittelt. Für das subjektive Empfinden der Anfaßklebrigkeit (Tack) wird eine hohe Frequenz und für die Anschmiegsamkeit eine niedrige Frequenz gewählt und die entsprechenden Quotienten über dem Schäumungsgrad ermittelt. Je höher der entsprechende Zahlenwert des Quotienten ist, desto besser ist die subjektive Eigenschaft.

Die Anfaßklebrigkeit und die Anschmiegsamkeit konnten erfindungsgemäß verbessert werden, wie in der Tabelle dargestellt.

$$Q = \tan \delta = G''/G'$$

| Bezeichnung | Anschmiegsamkeit niedrige Frequenz/RT | Anfaßklebrigkeit hohe Frequenz/RT |
|---|---|---|
| Schmelzhaftkleber A (nicht geschäumt) | $\tan \delta = 0{,}35 \pm 0{,}05$ | $\tan \delta = 0{,}45 \pm 0{,}05$ |
| Schmelzhaftkleber A Schaum Vol. ($N_2$)=50% | $\tan \delta = 0{,}46 \pm 0{,}05$ | $\tan \delta = 0{,}65 \pm 0{,}05$ |
| Schmelzhaftkleber A (nicht geschäumt) | $\tan \delta = 0{,}35 \pm 0{,}05$ | $\tan \delta = 0{,}45 \pm 0{,}05$ |
| Schmelzhaftkleber A Schaum Vol. ($N_2$) = 70% | $\tan \delta = 0{,}58 \pm 0{,}05$ | $\tan \delta = 0{,}88 \pm 0{,}05$ |
| Schmelzhaftkleber B (nicht geschäumt) | $\tan \delta = 0{,}05 \pm 0{,}03$ | $\tan \delta = 0{,}84 \pm 0{,}05$ |
| Schmelzhaftkleber B Schaum Vol. ($N_2$)=50% | $\tan \delta = 0{,}27 \pm 0{,}05$ | $\tan \delta = 1{,}15 \pm 0{,}05$ |
| Schmelzhaftkleber C (nicht geschäumt) | $\tan \delta = 0{,}06 \pm 0{,}03$ | $\tan \delta = 0{,}93 \pm 0{,}05$ |
| Schmelzhaftkleber C Schaum Vol. ($N_2$)=50% | $\tan \delta = 0{,}31 \pm 0{,}05$ | $\tan \delta = 1{,}25 \pm 0{,}05$ |

Es wurden verschiedene Schmelzhaftkleber, d.h. A auf Acrylat-, B und C auf Blockcopolymeren-Basis, gewählt und die Ergebnisse zeigen einen deutlichen Anstieg der tan δ-Werte durch die Schäumung, d.h. eine meßbar bessere Anschmiegsamkeit und Anfaßklebrigkeit.

Die geschilderten Vorteile machen die erfindungsgemäßen Trägermaterialien besonders geeignet für medizinische Zwecke. Daraus beispielsweise hergestellte Pflaster, Binden oder Bandagen oder zusätzlich mit einer Wundauflage versehene Wundverbände sind bei entsprechend ausgewähltem luftdurchlässigem Trägermaterial und einer hypoallergenen Klebemasse besonders gut hautverträglich, da sie über die ganze Fläche ausgeprägt luft- und wasserdampfdurchlässig sowie weich und anschmiegsam sind. Sie wirken wie gepolstert und weisen dadurch gute Trageeigenschaften auf bei gleichzeitig gutem Haftvermögen.

Auch kohäsive Haftbeschichtungen, d.h. Antirutschbeschichtungen, welche nur auf sich selbst kleben bzw. kaum klebenden Charakter aufweisen, können erfindungsgemäß hergestellt werden.

Sollen speziell geformte Pflaster hergestellt werden, kann die Klebemassenbeschichtung durch entsprechende Schablonen gleich produktentsprechend aufgedruckt und anschließend die Pflaster konturenscharf ausgestanzt werden.

Die vorteilhaften Eigenschaften der erfindungsgemäßen Klebebeschichtungen, wie geringer Klebstoffverbrauch, hohe Anfaßklebrigkeit und gute Anschmiegsamkeit auch an unebenen Flächen durch die Elastizität und Plastizität der geschäumten Klebemassen lassen sich auch auf rein technischem Gebiet nutzen. Die erhaltenen Selbstklebebänder und andere auf diese Weise selbstklebend ausgerüsteten Produkte lassen sich vielfältig einsetzen.

Ein besonders geeignetes Verfahren zur Herstellung der erfindungsgemäß selbstklebend ausgerüsteten Trägermaterialien arbeitet nach dem Schaum-Mix-System. Hierbei wird der thermoplastische Haftkleber unter hohem Druck bei ca. 120 Grad Celsius mit trockenen Gasen wie z.B. Stickstoff, Luft oder Kohlendioxid in unterschiedlichen Volumenanteilen (etwa 10-80%) in einem Stator/Rotorsystem umgesetzt. Während der Gasvordruck >100 bar ist, betragen die Mischdrucke Gas/Thermoplast im System 40-100 bar, bevorzugt 40-70 bar. Der so hergestellte Haftklebeschaum gelangt über eine Leitung in die Düsenzuführung einer Schmelz-Siebdruckbeschichtungsanlage.

Das Prinzip des Thermo-Rotationssiebdrucks besteht in der Verwendung einer rotierenden beheizten, nahtlosen, trommelförmigen perforierten Rundschablone, die über eine Düse mit der geschäumten Haftklebemasse beschickt wird. Eine speziell geformte Düsenlippe (Rund- oder Vierkantrakel) preßt die über einen Kanal eingespeiste Selbstklebemasse in Schaumform durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoff-Schaumkalotten geschieht dabei nach folgendem Mechanismus:

Der Düsenrakeldruck fördert die Schaumklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Schaumklebemasse und die innere Kohäsion des Hotmelts wird von der auf dem Träger bereits haftenden Basis der Kalotten der in den Löchern begrenzte Vorrat an Schaumklebemasse konturenscharf abgezogen bzw. durch den Rakeldruck auf den Träger gefördert.

Nach Beendigung dieses Schaumtransportes formt sich, abhängig von der Rheologie der Schaumklebemassse, über der vorgegebenen Basisfläche die mehr oder weniger stark gekrümmte Oberfläche der Schaumkalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Schaumklebemasse ab.

Der beschriebene Bildungsmechanismus der Schaumkalotten erfordert bevorzugt saugfähige oder zumindestens von Schaumklebemassen benetzbare Trägermaterialien. Nichtbenetzende Trägeroberflächen müssen durch chemische oder physikalische Verfahren vorbehandelt werden. Dies kann durch zusätzliche Maßnahmen wie z.B. Coronaentladung oder Beschichtung mit die Benetzung verbessernden Stoffen geschehen.

Mit dem aufgezeigten Druckverfahren kann die Größe und Form der Schaumkalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 - 2000 μm gewählt werden, die Höhe der Kalotten von 20 - ca. 2000 μm, bevorzugt 50 - 1000 μm, wobei der Bereich kleiner Durchmesser für glatte Träger, der mit größerem Durchmesser und größerer Kalottenhöhe für rauhe oder stark porige Trägermaterialien vorgesehen ist.

Die Positionierung der Schaumkalotten auf dem Träger wird durch die in weiten Grenzen variierbare Geometrie des Auftragswerkes, z.B. Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Es zeigte sich ferner, daß jeder Träger, gegebenenfalls nach Vorbehandlung, unabhängig von der Oberflächenrauhigkeit und Saugfähigkeit bedruckt werden kann, und das in sehr wirtschaftlicher Weise.

Mit dem Druck-Beschichtungsverfahren lassen sich viele Träger, sofern sie ausreichend wärmestabil sind, direkt beschichten. Universell einsetzbar ist es jedoch für alle flächigen und auch räumlichen Träger durch das sogenannte Transfer-Verfahren, bei welchem die Klebeschicht erst auf einen Zwischenträger aufgebracht wird und dann auf den endgültigen Träger übertragen wird.

Nachfolgend wird die Erfindung durch Beispiele näher erläutert:

Beispiel 1

Erfindungsgemäß wurde ein Augen-Occlusionspflaster hergestellt, welches für die Prophylaxe und Therapie des Strabismus und der Amblyopie bei Kindern eingesetzt wird. Dieses Pflaster besteht aus einem elastischen Baumwoll-Gewirke, welches mit einer Klebemasse auf Basis eines Blockcopolymeren im Thermosiebdruck beschichtet worden ist.

Bei dem Blockcopolymeren handelt es sich um ein Styrol-Ethylen-Butylen-Styrol-Blockcopolymerisat, welches mit Paraffinkohlenwasserstoffen versetzt worden ist. Das Verhältnis ist in diesem Fall zwei Teile Polymer zu einem Teil Paraffinkohlenwasserstoff. Der Kleber enthält 1% Alterungsschutzmittel (β-(3,5 Di-t.butyl-4-hydroxyphenyl)-propionsäure-n-octadecyclester), Handelsname: IRGANOX 1076) und weitere Kohlenwasserstoffharze und Fettsäureester, welche im Gesamtkleber nur zu geringen Mengen enthalten sind. Der Kleber ist handelsüblich erhältlich (Fa. Fuller). Die Klebemasse wurde nach dem beschriebenen Verfahren bei 120° C im Verhältnis 1:2 mit Stickstoff geschäumt. Der daraus resultierende Gasanteil im Endprodukt beträgt 50%. Auf der Klebeschicht von 30g/m$^2$ wurde mittig eine mehrschichtige Einlage, welche aus einer Abdeckfolie, Lichtschutzeinlage, Saugfolie und einer zusätzlichen Gitterfolie zur Abdeckung besteht, befestigt, wobei ein ausreichender Kleberand freigelassen wurde. Das komplette Pflaster wurde mit einem silikonisierten Trennpapier eingedeckt.

Die Klebemasse wurde durch Siebdruck auf dem Träger so appliziert, daß an den Rändern mehr Klebemasse war als in der Mitte des Pflasters. Die Beschichtungspunkte am Rand des Pflasters wurden möglichst groß gewählt, um die Haftung der Klebemasse auf dem Träger und eine hohe Klebesicherheit zu gewährleisten. In der Mitte des Pflasters wurde der Klebemasseauftrag nur so gering gewählt, daß die Einlage sicher auf dem Träger befestigt ist. Durch die konturenscharfe Bedruckung konnte weitere Klebemasse eingespart werden. Dieses ist durch die Auswahl der Siebschablone möglich. Nach der Beschichtung wurde das Pflaster entsprechend ausgestanzt. Die mit der Haut in Berührung kommende Klebeschicht ist ausgeprägt luft- und wasserdampfdurchlässig, die Anfaßklebrigkeit ist sehr gut. Das Pflaster ist durch die hohe Kohäsivität des Klebers und der damit verbundenen geringeren Epilation der Augenbraue leicht und fast schmerzfrei wieder abzuziehen.

Beispiel 2

Elastische Binden werden bisher in der Regel indirekt beschichtet. Hierbei wird die Klebemasse auf silikonbeschichtetes Trennpapier ausgestrichen und das Lösemittel im Trockenkanal entfernt. Anschließend wird das elastische Trägermaterial, ein Gewebe oder Gewirke, aufkaschiert. Eine derart hergestellte Binde ist jedoch nicht immer ausreichend luftdurchlässig.

Eine erfindungsgemäß hergestellte Binde wurde im Thermosiebdruck mit einem Masseauftrag von ca. 160g/m$^2$ Klebemasse auf Basis eines Blockcopolymeren beschichtet. Bei dem Blockcopolymeren handelt es sich um ein Styrol-Ethylen-Butylen-Styrol-Blockcopolymerisat, welches mit Paraffinkohlenwasserstoffen versetzt worden ist. Das Verhältnis ist in diesem Fall ein Teil Polymer zu einem Teil Paraffinkohlenwasserstoff. Zu der hergestellten Mischung wurden 10% Polystyrolharz (z.B. Amoco 18240) gegeben. Der Kleber enthält ferner ein Prozent Alterungsschutzmittel (β-(3,5 Di-t.butyl-4-hydroxy-phenyl)-propionsäure-n-octadecylester), Handelsname IRGANOX 1076) und weitere Kohlenwasserstoffharze und Fettsäureester, welche im Gesamtkleber nur zu geringen Mengen enthalten sind. Der Kleber ist handelsüblich erhältlich ( Fa. Fuller). Die Klebemasse wurde nach dem oben beschriebenen Verfahren bei 120° C im Verhältnis 1:2 mit Stickstoff geschäumt. Der daraus resultierende Gasanteil im Endprodukt betrug dann 50%.

Mit einer 14 Mesh Siebschablone und einem Duchlaß von 50% wurde der hohe Masseauftrag erreicht. Durch die Verwendung der großen Beschichtungspunkte konnte eine gute Haftung auf dem Träger und ein sauberes Schneiden erzielt werden. Durch die geschäumte, offenporige Klebemasse ist die Binde auch in einem mehrlagigen Verband luft- und wasserdampfdurchlässig. Sie wird für Kompressions-, Stütz- und Entlastungsverbände eingesetzt, wobei die hohe Sofort- und Dauerklebekraft vorteilhaft sind. Weiter unterstützt die Klebemasse auf Basis des Blockcopolymers durch die Elastizität des Klebers die kompressive Wirkung der Binde. Die Modellierbarkeit und Anwenderempfindung sind durch die Schäumung der Klebemasse verbessert worden.

Beispiel 3

Dieses Beispiel verdeutlicht den Einsatz der Erfindung für Wundverbände. Hierbei wird eine Folie aus Polyurethan mit einer Dicke von 60 μm im Thermosiebdruck mit einer geschäumten Acrylat-Hotmelt-Klebemasse beschichtet. Die Klebemasse wird gemäß der Stanzkontur auf die Folie direkt aufgetragen. Der Masseauftrag beträgt in der Randzone 60g/m$^2$ und in der Mittelzone 25 g/m$^2$. Die Mittelzone wird nach der Beschichtung mit einer Wundauflage, bevorzugt aus einem Vlies oder Schaum mit superabsorbierender Wirkung eingedeckt. Danach wird das Plaster wie üblich weiter konfektioniert und gegebenenfalls durch γ-Bestrahlung sterilisiert. Der Wundverband ist vollflächig atmungsaktiv und wasserdampfdurchlässig, wodurch die Haut vor Mazerationen geschützt wird und der Verband länger getragen werden kann. Weiter ist durch den hohen Klebemassenauftrag in den äußeren Randzonen eine zuverlässige Haftung erreicht worden.

Beispiel 4

Dieses Beispiel zeigt den Einsatz der Erfindung für einen Fingerkuppenverband. Hierbei wird ein Vlies aus Polyamid mit einer Dicke von 60μm im Thermosiebdruck mit einer geschäumten Styrol-Ethylen-Butylen-Styrol-Blockcopolymerisat-Hotmelt-Klebemasse beschichtet. Die Klebemasse wird gemäß der Stanzkontur auf das Vlies direkt aufgetragen. Der Masseauftrag beträgt in der Randzone 60g/m$^2$ und in der Mittelzone 25g/m$^2$. Die Mittelzone wird nach der Beschichtung mit einer Wundauflage, bevorzugt aus einem Vlies oder Schaum mit superabsorbierender Wirkung, versehen und das Pflaster nach Eindeckung mit der üblichen Schutzabdeckung ausgestanzt und verpackt.

## Patentansprüche

1. Selbstklebend ausgerüstetes Trägermaterial mit einer mindestens auf einer Seite nicht vollflächig aufgebrachten Heißschmelzselbstklebemasse, dadurch gekennzeichnet, daß die Klebemasse geschäumt ist.

2. Selbstklebend ausgerüstetes Trägermaterial gemäß Anspruch 1, dadurch gekennzeichnet, daß die geschäumte Klebemasse rasterförmig durch Siebdruck oder Tiefdruck aufgebracht ist.

3. Selbstklebend ausgerüstetes Trägermaterial gemäß Anspruch 1, dadurch gekennzeichnet, daß die geschäumte Klebemasse aufgesprüht ist.

4. Selbsklebend ausgerüstetes Trägermaterial gemäß Anspruch 1, 2 oder 3, dadurch gekennzeichnet, daß die Klebemasse funktionsgerecht für das Produkt über die Fläche unterschiedlich stark und/oder dicht aufgetragen ist.

5. Selbstklebend ausgerüstetes Trägermaterial gemäß einem oder mehreren der vorangehenden Ansprüche, dadurch gekennzeichnet, daß die Klebemasse mit einer Flächendeckung von 20-95% aufgebracht ist.

6. Selbstklebend ausgerüstetes Trägermaterial gemäß einem oder mehreren der vorangehenden Ansprüche, dadurch gekennzeichnet, daß die Klebemasse mit einem inerten Gas wie Stickstoff, Kohlendioxid, Edelgasen, Kohlenwasserstoffe oder Luft oder deren Gemische geschäumt ist.

7. Selbstklebend ausgerüstetes Trägermaterial gemäß einem oder mehreren der vorangehenden Ansprüche, dadurch gekennzeichnet, daß der Gasanteil in der Klebemasse 10-80 Volumenprozent, vorzugsweise etwa 30-70%, beträgt.

8. Selbstklebend ausgerüstetes Trägermaterial gemäß einem oder mehreren der vorangehenden Ansprüche, dadurch gekennzeichnet, daß die Klebemasse auf A-B-A Blockcopolymer-Basis aufgebaut ist, wobei A Polystyrol und B Ethylen, Propylen, Butylen, Butadien, Isopren oder deren Mischungen wie Ethylen/Butylen bedeuten.

9. Verfahren zur Herstellung von selbstklebend ausgerüstetem Trägermaterial gemäß einem oder mehreren der vorangehenden Ansprüche, dadurch gekennzeichnet, daß die Heißschmelzselbstklebemassen unter hohem Druck mit inerten Gasen in Volumenanteilen von 10-80% in einem Stator/Rotorsystem umgesetzt werden, der so hergestellte Haftklebeschaum in die Düsenzuführung einer Schmelz-Siebdruck-Rotationsbeschichtungsvorrichtung geleitet wird und von dieser auf das Trägermaterial appliziert wird.

10. Verfahren gemäß Anspruch 9, dadurch gekennzeichnet, daß die Klebemasse in Form von kleinen Kalotten auf das Trägermaterial aufgebracht wird.

11. Verwendung eines selbstklebend ausgerüsteten Trägermaterials gemäß einem oder mehreren derAnsprüche 1 bis 8 für medizinische Produkte.

12. Verwendung gemäß Anspruch 11, wobei die medizinischen Produkte Heftpflaster, Fixierpflaster, Testpflaster, Wundschnellverbände, Binden, orthopädische Binden und Bandagen, Wundauflagen, Inzisionsfolien oder Colostomiebeutel sind.

**Claims**

1. Carrier material having a self-adhesive finish and a hotmelt self-adhesive composition applied not to the entire area of at least one side, characterized in that the adhesive composition is foamed.

2. Carrier material having a self-adhesive finish according to Claim 1, characterized in that the foamed adhesive composition is applied in a pattern by screen printing or intaglio printing.

3. Carrier material having a self-adhesive finish according to Claim 1, characterized in that the foamed adhesive composition is sprayed on.

4. Carrier material having a self-adhesive finish according to Claim 1, 2 or 3, characterized in that the adhesive composition is applied over the surface in varying thicknesses and/or densities as appropriate for the function of the product.

5. Carrier material having a self-adhesive finish according to one or more of the preceding claims, characterized in that the adhesive composition is applied with a surface coverage of 20-95 %.

6. Carrier material having a self-adhesive finish according to one or more of the preceding claims, characterized in that the adhesive composition is foamed with an inert gas such as nitrogen, carbon dioxide, noble gases, hydrocarbons or air, or mixtures thereof.

7. Carrier material having a self-adhesive finish according to one or more of the preceding claims, characterized in that the proportion of gas in the adhesive composition is 10-80 percent by volume, preferably about 30-70 %.

8. Carrier material having a self-adhesive finish according to one or more of the preceding claims, characterized in that the adhesive composition is composed on an A-B-A block copolymer basis, A being polystyrene and B being ethylene, propylene, butylene, butadiene, isoprene or mixtures thereof such as ethylene/butylene.

9. Process for producing carrier material having a self-adhesive finish according to one or more of the preceding claims, characterized in that the hotmelt self-adhesive compositions are reacted under high pressure with inert gases in proportions by volume of 10-80 % in a stator/rotor system, the pressure-sensitive adhesive foam produced in this way is passed into the supply nozzle of a melt-screen printing rotary coating device, and is applied thereby to the carrier material.

10. Process according to Claim 9, characterized in that the adhesive composition is applied in the form of small domes to the carrier material.

11. Use of a carrier material having a self-adhesive finish according to one or more of Claims 1 to 8 for medical products.

12. Use according to Claim 11, the medical products being sticking plasters, fixing plasters, test plasters, rapid dressings, bandages, orthopaedic bandages, wound pads, incision films or colostomy bags.

**Revendications**

1. Matériau de support avec un apprêt autocollant et une composition autocollante thermofusible non appliquée sur toute la surface d'au moins une face, caractérisé en ce que la composition adhésive est expansée.

2. Matériau de support avec un apprêt autocollant selon la revendication 1, caractérisé que la composition adhésive expansée est appliquée avec un motif quadrillé par sérigraphie ou héliogravure.

**3.** Matériau de support avec un apprêt autocollant selon la revendication 1, caractérisé en ce que la composition adhésive expansée est pulvérisée.

**4.** Matériau de support avec un apprêt autocollant selon la revendication 1, 2 ou 3, caractérisé en ce que la composition adhésive est appliquée sur la surface avec des épaisseurs et/ou des densités variables conformément à la fonction du produit.

**5.** Matériau de support avec un apprêt autocollant selon l'une ou plusieurs des revendications précédentes, caractérisé en ce que la composition adhésive est appliquée avec un recouvrement de surface de 20 à 95%.

**6.** Matériau de support avec un apprêt autocollant selon l'une ou plusieurs des revendications précédentes, caractérisé en ce que la composition adhésive est expansée avec un gaz inerte tel que l'azote, le dioxyde de carbone, des gaz nobles, des hydrocarbures ou l'air, ou leurs mélanges.

**7.** Matériau de support avec un apprêt autocollant selon l'une ou plusieurs des revendications précédentes, caractérisé en ce que la proportion de gaz dans la composition adhésive est de 10 à 80 pour cent en volume, de préférence d'environ 30 à 70%.

**8.** Matériau de support avec un apprêt autocollant selon l'une ou plusieurs des revendications précédentes, caractérisé en ce que la composition adhésive est constituée à base d'un copolymère bloc A-B-A, A représentant le polystyrène et B représentant l'éthylène, le propylène, le butylène, le butadiène, l'isoprène ou leurs mélanges tel que l'éthylène/butylène.

**9.** Procédé de production d'un matériau de support avec un apprêt autocollant selon l'une ou plusieurs des revendications précédentes, caractérisé en ce que les compositions autocollantes thermofusibles sont mises à réagir sous une pression élevée avec des gaz inertes en proportions volumiques de 10 à 80% dans un système de stator/rotor, la mousse adhésive autocollante ainsi préparée est passée dans l'unité d'alimentation de la buse d'un dispositif de revêtement rotatif à fusion par sérigraphie et est appliquée sur le matériau de support à partir de celle-ci.

**10.** Procédé selon la revendication 9, caractérisé en ce que la composition adhésive est appliquée sous forme de petites calottes sphériques sur le matériau de support.

**11.** Utilisation d'un matériau de support avec un apprêt autocollant selon l'une ou plusieurs des revendications 1 à 8 pour des produits médicaux.

**12.** Utilisation selon la revendication 11, dans laquelle les produits médicaux sont des sparadraps, des emplâtres de fixation, des emplâtres d'essai, des pansements rapides, des bandes, des bandes et bandages orthopédiques, des tampons vulnéraires, des films d'incision ou des poches pour colostomie.